(19) Europäisches Patentamt

European Patent Office

Office européen des brevets

(11) EP 1 695 690 A1

(12) DEMANDE DE BREVET EUROPEEN

(43) Date de publication:
30.08.2006 Bulletin 2006/35

(51) Int Cl.:
A61K 8/06 (2006.01)        A61K 8/891 (2006.01)
A61Q 1/14 (2006.01)

(21) Numéro de dépôt: 05027826.6

(22) Date de dépôt: 20.12.2005

(84) Etats contractants désignés:
AT BE BG CH CY CZ DE DK EE ES FI FR GB GR
HU IE IS IT LI LT LU LV MC NL PL PT RO SE SI
SK TR
Etats d'extension désignés:
AL BA HR MK YU

(30) Priorité: 31.01.2005  FR 0550263

(71) Demandeur: L'ORÉAL
75008 Paris (FR)

(72) Inventeur: Bonafos, Arnaud
75014 Paris (FR)

(74) Mandataire: Rasson, Catherine
L'OREAL - D.I.P.I.
25-29 Quai Aulagnier
92600 Asnières (FR)

(54) **Composition démaquillante**

(57) L'invention se rapporte à une composition cosmétique sous forme d'émulsion huile-dans-eau, caractérisée en ce qu'elle comprend au moins un tensioactif siliconé, au moins une huile et au moins un tensioactif détergent.

L'addition du tensioactif siliconé permet d'améliorer notablement le pouvoir démaquillant de la composition tout en ne laissant aucune sensation de gras ou de collant sur la peau.

La composition peut se présenter sous forme d'un lait ou d'une crème.

**Description**

La présente invention se rapporte à une composition cosmétique sous forme d'émulsion huile-dans-eau (H/E), comportant un tensioactif siliconé, et à l'utilisation de cette composition pour nettoyer ou démaquiller la peau du visage et/ou du corps. En particulier, cette émulsion peut constituer un lait démaquillant ou une crème démaquillante.

L'invention a également pour objet un procédé cosmétique pour nettoyer ou démaquiller la peau.

Les démaquillants et nettoyants du visage se présentant sous forme d'émulsions, classiquement utilisés à ce jour, présentent des inconvénients qui ne sont pas les mêmes selon le sens de l'émulsion. Dans le cas des émulsions eau-dans-huile (E/H), très efficaces pour le démaquillage/nettoyage du fait que la phase grasse externe est directement disponible pour dissoudre les différents corps gras présents sur la peau ou issus du maquillage, leur utilisation est inconfortable du fait de la sensation grasse et lourde apportée par cette phase grasse externe qui subsiste sur la peau.

Dans le cas des émulsions huile-dans-eau (H/E), la phase aqueuse externe apporte de la fraîcheur. En revanche, la phase huileuse étant la phase interne, elle n'est pas directement disponible pour dissoudre les corps gras et, de ce fait, le démaquillage est moins efficace. Or, pour le nettoyage du visage et plus spécialement pour le démaquillage qui consiste à enlever tous les produits de maquillage, les femmes cherchent à obtenir un démaquillage le plus efficace possible.

Pour améliorer l'efficacité d'une composition démaquillante sous forme d'émulsion H/E, il est nécessaire d'introduire une quantité importante de phase grasse (phase huileuse), puisque c'est cette dernière qui assure une bonne dissolution des particules solides qui composent le maquillage. Toutefois, quand on introduit une quantité importante d'huiles dans une telle composition, les qualités cosmétiques et la tolérance de la composition se trouvent compromises, car les compositions contenant une trop grande quantité d'huile, par exemple 30 %, laissent un film gras sur la peau et donnent à la peau un aspect brillant et collant, ce qui est souvent rédhibitoire pour l'utilisatrice.

Il subsiste donc le besoin d'une émulsion démaquillante ayant une bonne efficacité tout en étant bien tolérée et en ayant de bonnes propriétés cosmétiques, c'est-à-dire n'ayant ni effet gras ni effet collant ni effet brillant.

La demanderesse a trouvé de manière surprenante qu'il était possible d'obtenir une composition démaquillante sous forme d'émulsion H/E contenant beaucoup d'huile, en utilisant un tensioactif siliconé.

La présente invention a donc pour objet une composition cosmétique sous forme d'émulsion huile-dans-eau, comportant une phase huileuse dispersée dans une phase aqueuse, caractérisée en ce qu'elle comprend au moins un tensioactif siliconé, au moins une huile et au moins un tensioactif détergent.

Cette composition cosmétique constitue notamment une composition nettoyante ou démaquillante.

L'émulsion obtenue selon l'invention a la propriété non seulement d'être non irritante et stable, mais encore d'être très efficace tout en étant agréable à utiliser, en particulier en étant d'une grande douceur à l'application, et en ne laissant aucune sensation de gras ou de collant.

La demanderesse a constaté de manière surprenante que les tensioactifs siliconés augmentaient l'efficacité de démaquillage des huiles présentes dans l'émulsion. Le résultat obtenu est d'autant plus surprenant que les tensioactifs siliconés sont connus pour être des émulsionnants des émulsions E/H alors qu'il s'agit ici d'une émulsion H/E et que l'émulsion revendiquée ne contient pas d'autre émulsionnant puisque les autres tensioactifs qu'elle contient sont des tensioactifs détergents et non des tensioactifs émulsionnants.

Aussi, la présente invention a aussi pour objet l'utilisation d'au moins un tensioactif siliconé pour améliorer l'efficacité de démaquillage d'une émulsion huile-dans-eau contenant au moins une huile et au moins un tensioactif détergent.

La composition cosmétique étant une composition pour application topique, elle comprend un milieu physiologiquement acceptable. On entend dans la présente demande par « milieu physiologiquement acceptable », un milieu compatible avec toutes les matières kératiniques telles que la peau y compris le cuir chevelu, les ongles, les muqueuses, les yeux et les cheveux ou toute autre zone cutanée du corps.

Par ailleurs, on entend par « tensioactif détergent », un tensioactif inapte à stabiliser seul à long terme une émulsion, mais ayant des propriétés détergentes, c'est-à-dire apte à donner un effet nettoyant ou détergent. Généralement de tels tensioactifs ont un HLB (Hydrophilic Lipophilic balance) assez élevé. Le terme HLB est bien connu de l'homme du métier et est décrit dans "The HLB system. A time-saving guide to Emulsifier Selection" (published by ICI Americas Inc ; 1984). De préférence, le tensioactif détergent utilisé ou le mélange de tensioactifs détergents utilisés a un HLB égal ou supérieur à 11, par exemple allant de 11 à 18, et de préférence de 12 à 17.

Tensioactif siliconé

Les tensioactifs siliconés utilisés dans la présente invention sont des tensioactifs émulsionnants, et ce sont des polydiméthylsiloxanes comportant des groupes oxyéthylénés et/ou oxypropylénés. On utilise de préférence ceux comportant une chaîne oxyéthylénée et/ou oxypropylénée suivante :

$$-(C_2H_4O)_x-(C_3H_6O)_y-H$$

où x peut aller de 0 à 100 et y peut aller de 0 à 100, x et y ne pouvant pas être tous deux 0.

Selon un mode préféré de réalisation de l'invention, ces tensioactifs siliconés comportent à la fois des groupes oxyéthylénés et des groupes oxypropylénés, par exemple de 2 à 50 groupes oxyéthylénés et de 2 à 50 groupes oxypropylénés. Les tensioactifs siliconés utilisés dans la composition de l'invention peuvent être choisis parmi les dimethicone copolyols et les alkyldiméthicone copolyols.

Les dimethicone copolyols utilisables dans la composition de l'invention peuvent être choisis notamment parmi ceux comportant de 2 à 50 groupes oxyéthylénés et de 2 à 50 groupes oxypropylénés, comme par exemple celui comportant 18 groupes oxyéthylénés et 18 groupes oxypropylénés (PEG/PPG-18/18 Dimethicone), tels que le mélange de dimethicone copolyol comportant 18 groupes oxyéthylénés et 18 groupes oxypropylénés, de cyclopentasiloxane et d'eau (10/88/2), commercialisé par la société Dow Corning sous la dénomination DC-3225C ou DC2-5225C (nom INCI: Cyclopentasiloxane / PEG/PPG-18/18 Dimethicone), ou celui comportant 14 groupes oxyéthylénés et 14 groupes oxypropylénés (PEG/PPG-14/14 Dimethicone) tels que, notamment, le mélange de dimethicone copolyol comportant 14 groupes oxyéthylénés et 14 groupes oxypropylénés et de cyclopentasiloxane (85/15) commercialisé sous la dénomination Abil EM-97 par la société Goldschmidt (nom INCI : Bis-PEG/PPG-14/14 Dimethicone / Cyclopentasiloxane).

Comme alkyldiméthicone copolyols, on peut citer notamment ceux ayant un radical alkyle comportant de 10 à 22 atomes de carbone et comportant de 2 à 50 groupes oxyéthylénés et de 2 à 50 groupes oxypropylénés, tel que le cétyl diméthicone copolyol (nom INCI: Cetyl PEG/PPG-10/1 Dimethicone) comme le produit commercialisé sous la dénomination Abil EM-90 par la société Goldschmidt ; le lauryl diméthicone copolyol (nom INCI : Lauryl PEG/PPG-18/18 Methicone) et par exemple le mélange d'environ 91 % de lauryl diméthicone copolyol et d'environ 9 % d'alcool isostéarylique, commercialisé sous la dénomination DOW CORNING 5200 FORMULATION AID par la société Dow Corning.

On peut aussi utiliser un mélange de ces tensioactifs siliconés.

Selon un mode de réalisation particulièrement préféré, le tensioactif siliconé est choisi parmi les dimethicone copolyols, et notamment celui comportant 18 groupes oxyéthylénés et 18 groupes oxypropylénés, plus particulièrement le produit vendu sous la dénomination DC-5225 C.

La quantité de tensioactif siliconé dans la composition de l'invention, en matière active, peut aller par exemple de 0,01 à 5 % en poids, de préférence de 0,05 à 3 % en poids et mieux de 0,05 à 2 % en poids par rapport au poids total de la composition.

Phase huileuse

La phase huileuse peut constituer de 3 à 70 % en poids, de préférence de 5 à 60 % en poids et mieux de 10 à 50 % en poids par rapport au poids total de la composition.

La phase huileuse contient les huiles et autres corps gras et les additifs lipophiles. Elle contient de préférence au moins une huile et notamment au moins une huile démaquillante choisie parmi les huiles hydrocarbonées, les esters gras comportant au moins 8 atomes de carbone, et leurs mélanges.

On entend par « huile hydrocarbonée » toute huile comportant majoritairement des atomes de carbone et d'hydrogène. Comme huiles hydrocarbonées, on peut citer par exemple les huiles de paraffine, volatiles ou non, et leurs dérivés, l'huile de vaseline, les polydécènes, le polyisobutène hydrogéné tel que celui commercialisé sous la dénomination huile de Parléam®, l'isoparaffine hydrogénée, l'isohexadecane, l'isododecane, et leurs mélanges.

On entend par "ester gras" un ester obtenu à partir d'un acide gras et/ou à partir d'un alcool gras. Les esters gras comportent au moins 8 atomes de carbone et de préférence de 9 à 26 atomes de carbone et mieux de 12 à 22 atomes de carbone. L'ester constituant l'huile démaquillante peut être notamment choisi parmi :

(1) les esters obtenus à partir d'acide gras à chaîne droite ou ramifiée comportant au moins 9 atomes de carbone et d'alcool à chaîne droite ou ramifiée comportant de 1 à 17 atomes de carbone ;
(2) les esters obtenus à partir d'acide à chaîne droite ou ramifiée comportant 1 à 17 atomes de carbone et d'alcool gras à chaîne droite ou ramifiée comportant au moins 9 atomes de carbone ;
(3) les esters obtenus à partir de l'acide benzoïque et d'alcool gras à chaîne droite ou ramifiée comportant au moins 9 atomes de carbone ; et
(4) leurs mélanges.

Les esters sont de préférence de mono- ou di-esters.

De manière préférée, l'ester pouvant constituer l'huile démaquillante est choisi dans le groupe des esters qui ne comportent aucune insaturation et/ou aucun groupement éther ou hydroxyle. De façon encore plus avantageuse, il s'agit d'un ester saturé qui ne renferme aucun groupement éther ni hydroxyle.

Ainsi, l'ester pouvant être utilisé comme huile démaquillante dans la composition conforme à l'invention peut être notamment choisi dans le groupe comprenant le palmitate d'éthyl-2 hexyle (ou palmitate d'octyle), l'isononanoate d'isononyle, le lactate d'isostéaryle, le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, l'ester d'acide benzoïque et d'alcools en $C_{12}$-$C_{15}$ (nom INCI : C12-15 Alkyl Benzoate), et leurs mélanges.

La ou les huiles démaquillantes peuvent constituer tout ou une partie de la phase huileuse, et par exemple la quantité d'huile(s) démaquillante(s) peut aller de 0,5 à 70 %, de préférence 2 à 60 %, et mieux de 5 à 50 % en poids par rapport au poids total de la composition.

La phase huileuse peut contenir en outre tous les corps gras et notamment les huiles autres que celles indiquées ci-dessus, classiquement utilisés dans le domaine cosmétique. Comme autres huiles susceptibles d'être présentes dans la phase huileuse, on peut citer par exemple les huiles d'origine végétale comme l'huile de noyaux d'abricot ou l'huile d'amande douce ; les huiles de synthèse ; les huiles de silicone volatiles ou non volatiles et les huiles fluorées.

Comme huiles de synthèse, on peut citer par exemple l'huile de Purcellin ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle.

Comme huiles de silicone, on peut citer par exemple les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; leurs mélanges.

Les autres corps gras susceptibles d'être présents dans la phase huileuse peuvent être par exemple les acides gras, les alcools gras tels que l'alcool cétylique, l'alcool stéarylique et leurs mélanges (alcool cétéarylique), la vaseline (petrolatum) et les cires.

Phase aqueuse

La composition selon l'invention comporte une quantité de phase aqueuse qui peut aller par exemple de 30 à 97 % en poids, de préférence de 40 à 95 % en poids et mieux de 50 à 90 % en poids par rapport au poids total de la composition.

La phase aqueuse comprend de l'eau et tout autre composé hydrosoluble éventuellement présent, tels que notamment les solvants et additifs hydrosolubles (par exemple tensioactifs hydrophiles et actifs).

Comme solvants hydrosolubles, on peut citer notamment les alcools inférieurs et les polyols. On entend par alcool inférieur, les alcools comportant de 1 à 8 et plus particulièrement de 1 à 6 atomes de carbone, tels que l'éthanol. Comme polyols, on peut citer par exemple la glycérine, le propylène glycol, le butylène glycol et les polyéthylène glycols (PEG-8 par exemple). Ces alcools et/ou polyols peuvent être présents dans la composition en une quantité allant de préférence de 0,1 à 25 % et de 1 à 15 % du poids total de la composition.

Tensioactifs détergents

Les tensioactifs détergents sont des tensioactifs qui vont apporter le caractère nettoyant à la composition. Ces tensioactifs peuvent être choisis parmi les tensioactifs détergents non ioniques, anioniques, amphotères, zwitterioniques et leurs mélanges. Comme indiqué ci-dessus, le tensioactif détergent utilisé ou le mélange de tensioactifs détergents utilisés a généralement un HLB (Hydrophilic lipophilic balance) égal ou supérieur à 11.

La quantité de tensioactif(s) détergent(s) peut aller par exemple, en poids de matière active, de 0,05 à 10 % en poids, de préférence de 0,1 à 5 % en poids et mieux de 0,2 à 2 % en poids par rapport au poids total de la composition.

Comme tensioactifs détergents, on peut citer :

1. Les tensioactifs non ioniques :

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle à chaîne droite comportant de 6 à 30 atomes de carbone et de préférence de 8 à 16 atomes de carbone, et contenant un groupe hydrophile (glucoside) comprenant de préférence 1,2 à 3 unités de glucoside. Comme alkylpolyglucosides, on peut citer par exemple le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP® par la société Cognis, et le produit commercialisé sous la dénomination ORAMIX NS 10 ® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N ® et PLANTACARE 1200 ® par la société Cognis ; et le cocoglucoside comme le produit commercialisé sous la dénomination PLANTACARE 818/UP ® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés, on peut citer le dodecanediol polyglycérolé (3,5 moles de glycérol), produit commercialisé sous la dénomination CHIMEXANE NF® par la société Chimex.

2. Les tensioactifs anioniques.

Ces tensioactifs peuvent être choisis notamment parmi les carboxylates, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkyl polyglucoside, les savons d'acides gras, et leurs mélanges.

Comme carboxylates, on peut citer par exemple les sels alcalins de N-acylaminoacides ; les amido éthercarboxylates (AEC) comme le lauryl amido ether carboxylate de sodium (3 OE) commercialisé sous la dénomination AKYPO FOAM 30® par la société Kao Chemicals ; les sels d'acides carboxyliques polyoxyéthylénés, comme le lauryl ether carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxymethyl, produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX ® par la société Nikkol.

Les dérivés des aminoacides peuvent être choisis par exemple parmi les sarcosinates et notamment les acylsarcosinates comme le lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium, commercialisé sous la dénomination NIKKOL SARCOSINATE MN® par la société Nikkol, le palmitoyl sarcosinate de sodium, commercialisé sous la dénomination NIKKOL SARCOSINATE PN ® par la société Nikkol ; les alaninates comme le N-lauroyl-N-methylamidopropionate de sodium, commercialisé sous la dénomination SODIUM NIKKOL ALANINATE LN 30® par la société Nikkol ou commercialisé sous la dénomination ALANONE ALE®,par la société Kawaken, et le N-lauroyl N-methylalanine triéthanolamine, commercialisé sous la dénomination ALANONE ALTA ® par la société Kawaken ; les N-acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; les aspartates comme le mélange de N-lauroylaspartate de triéthanolamine et de N-myristoylaspartate de triéthanolamine, commercialisé sous la dénomination ASPARACK ® par la société Mitsubishi ; les citrates, et leurs mélanges.

Comme alkyl sulfates, on peut citer par exemple le lauryl sulfate de triéthanolamine (nom INCI : TEA-lauryl sulfate) tel que le produit commercialisé par la société Huntsman sous la dénomination EMPICOL TL40 FL ou celui commercialisé par la société Cognis sur la dénomination TEXAPON T42, produits qui sont à 40% en solution aqueuse. On peut aussi citer le lauryl sulfate d'ammonium (nom CFTA : Ammonium lauryl sulfate) tel que le produit commercialisé par la société Huntsman sous la dénomination EMPICOL AL 30FL qui est à 30% en solution aqueuse.

Comme alkyl éther sulfates, on peut citer par exemple le lauryl éther sulfate de sodium (nom INCI : sodium laureth sulfate) comme celui commercialisé sous les dénominations TEXAPON N 40 et TEXAPON AOS 225 UP par la société Cognis, le lauryl éther sulfate d'ammonium (nom INCI : ammonium laureth sulfate) comme celui commercialisé sous la dénomination STANDAPOL EA-2 par la société Cognis.

Comme sulfonates, on peut citer par exemple les alpha-oléfines sulfonates comme l'alpha-oléfine sulfonate de sodium (C14-16) commercialisé sous les dénominations BIO-TERGE AS-40® et BIO-TERGE AS-40 CG® par la société Stepan, ou sous les dénominations WITCONATE AOS PROTEGE® et SULFRAMINE AOS PH 12® par la société Witco ; l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.

Comme iséthionates, on peut citer les acyliséthionates comme le cocoyl-iséthionate de sodium, tel que le produit commercialisé sous la dénomination JORDAPON CI P® par la société Jordan.

Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco.

Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR® par la société Croda, le sel de triéthanolamine de cocoyl protéine de soja hydrolysée, commercialisé sous la dénomination MAY-TEIN SY® par la société Maybrook, le sel de sodium des lauroyl amino-acides d'avoine, commercialisé sous la dénomination PROTEOL OAT® par la société Seppic, l'hydrolysat de collagène greffé sur l'acide gras de coprah, commercialisé sous la dénomination GELIDERM 3000® par la société Deutsche Gelatine, les protéines de soja acylées par des acides de coprah hydrogénés, commercialisées sous la dénomination PROTEOL VS 22® par la société Seppic.

Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel di-sodique d'ester sulfosuccinique de cocoylpolyglucoside (1,4), commercialisé sous la dénomination ESSAI 512 MP® par la société Seppic, le sel de sodium d'ester citrique de cocoyl polyglucoside (1,4) commercialisé sous la dénomination EUCAROL AGE-EC® par la société Cesalpinia.

Les savons d'acide gras qui peuvent être utilisés comme tensioactifs anioniques sont des acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les métaux alcalins ou alcalino-terreux, les aminoacides, et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique, stéarique (laurate, myristate, palmitate et stéarate de potassium ou de sodium), et leurs mélanges.

3. Les tensioactifs amphotères et zwitterioniques

Ces tensioactifs peuvent être choisis par exemple parmi les bétaïnes, les N-alkylamidobétaïnes et leurs dérivés, les dérivés de la glycine, les sultaïnes, les alkyl polyaminocarboxylates, les alkylamphoacétates et leurs mélanges.

Comme bétaïnes, on peut citer par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER (10 OE)BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou commercialisée sous la dénomination EMPIGEN BB® par la société Albright & Wilson, la lauramidopropyl bétaïne commercialisée sous la dénomination REWOTERIC AMB12P® par la société Witco.

Comme dérivés de la glycine, on peut citer le N-cocoylglycinate de sodium commercialisé sous la dénomination AMILITE GCS-12® par la société Ajinomoto.

Comme sultaines, on peut citer le cocoyl-amidopropylhydroxy-sulfobetaine commercialisé sous la dénomination CROSULTAINE C-50® par la société Croda.

Comme alkylpolyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom INCI : disodium cocoamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom INCI : sodium cocamphoacetate).

Parmi les tensioactifs cités ci-dessus, selon un mode particulier de réalisation de l'invention, on utilise plus particulièrement un ou plusieurs tensioactifs détergents choisis parmi les alkylpolyglucosides, les alkyl éther sulfates, les N-acylglutamates, les acyliséthionates, les sulfosuccinates, les phosphates et alkylphosphates, les bétaïnes, les alkylamphoacétates, et leurs mélanges.

Les tensioactifs détergents sont généralement introduits dans la phase aqueuse.

La composition selon l'invention a généralement un pH allant de 3 à 9, mieux de 4 à 9, et mieux de 4,5 à 8 et encore mieux de 5,5 à 8. Si besoin est, le pH de la composition est ajusté par addition d'un acide tel que l'acide citrique ou d'une base telle que l'hydroxyde de sodium ou la triéthanolamine.

Les compositions de l'invention constituent notamment des compositions cosmétiques de nettoyage ou de démaquillage. Elles ne sont pas moussantes et ne sont pas nécessairement rincées.

Les compositions de l'invention peuvent être plus ou moins fluides et elles peuvent constituer notamment un lait ou une crème, celle-ci étant plus ou moins épaisse.

Ainsi, selon un des modes particuliers de réalisation de l'invention, les compositions se présentent sous forme d'un lait, c'est-à-dire d'une composition assez fluide pour pouvoir s'écouler d'un flacon sous son propre poids. On entend par « composition fluide » ou « lait » une composition ayant une viscosité allant par exemple de 1 à 500 cP, soit 0,001 Pas à 0,5 Pas, et mieux allant de 1 à 100 cP (soit de 0,001 à 0,1 Pa.s), cette viscosité étant mesurée à la température ambiante (environ 25°C) à l'aide d'un Rhéomat RM180. Les laits peuvent être utilisés directement sur la peau mais le plus souvent par application d'un peut de lait sur un coton ou tout autre support approprié, et passage du coton sur la peau.

Selon un autre mode de réalisation de l'invention, les compositions se présentent sous forme de crème, ce qui correspond à une texture plus épaisse qu'un lait. Les crèmes ne s'écoulent pas sous leur propre point et sont généralement en pot alors que les laits sont dans des flacons. Les crèmes peuvent par exemple avoir une viscosité à 25°C allant d'environ 10 à 150 poises (1 à 15 Pa.s) et de préférence d'environ 15 à 100 poises (1,5 à 10 Pa.s), cette viscosité étant mesurée avec un appareil Rhéomat 180 à 25°C avec les mobiles appropriés (3 ou 4 selon la viscosité).

Les crèmes obtenues selon l'invention ont l'avantage de pouvoir contenir beaucoup d'huile et d'avoir une texture douce et onctueuse, et d'être donc très agréables à utiliser. De manière préférée, de telles crèmes sont présentées en pot et sont utilisées de la manière suivante : on prélève une noisette de crème qu'on applique avec les doigts sur le visage maquillé sec. En massant, la crème se transforme en huile et dissout le maquillage. On essuie ensuite le visage avec un mouchoir en papier ou tissu de type kleenex sec ou humide. La peau est nettoyée sans être agressée ; elle est en outre nourrie et hydratée, et le produit laisse une peau douce et non-grasse. Les avantages de ces crèmes sont leur consistance dense et riche, leur aspect onctueux, leur facilité de préhension dans le pot, leurs qualités de fraîcheur à l'application et leur efficacité de démaquillage.

De façon connue, la composition de l'invention peut en outre contenir un ou plusieurs adjuvants choisis parmi les adjuvants habituels dans le domaine cosmétique, tels que les actifs hydrophiles ou lipophiles, les conservateurs, les antioxydants, les parfums, les solvants, les charges, les fibres, les filtres solaires, les matières colorantes (pigments ou nacres), les ajusteurs de pH (bases telles que l'hydroxyde de sodium ou acides tels que l'acide citrique), les vésicules lipidiques, les polymères autres que ceux indiqués ci-dessus et leurs mélanges. Ces adjuvants sont utilisés dans les proportions habituelles dans le domaine cosmétique, par exemple de 0,01 à 20 % du poids total de la composition, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour la composition.

Selon la fluidité de la composition que l'on souhaite obtenir, on peut incorporer dans la composition, un ou plusieurs gélifiants, notamment hydrophiles, c'est-à-dire solubles ou dispersibles dans l'eau. Comme gélifiants hydrophiles, on peut citer en particulier les polymères épaississants hydrosolubles ou hydrodispersibles. Ceux-ci peuvent notamment être choisis parmi : les polymères carboxyvinyliques, tels que les produits commercialisés sous les dénominations Carbopol (nom INCI: carbomer) ; les polymères carboxyvinyliques comportant au moins un groupe hydrophobe, tels que ceux commercialisés sous les dénominations Pemulen TR1 ou Pemulen TR2 ou Carbopol 1342 (nom INCI : Acrylates/C10-30 akyl acrylate crosspolymer) par la société Noveon ; les polyacrylates et polyméthacrylates tels que les produits vendus sous les dénominations de Lubrajel et Norgel par la société Guardian ou sous la dénomination Hispagel par la société Hispano Chimica ou les polyacrylates de sodium réticulés tels que le produit commercialisé par la société Cognis sous le dénomination Cosmedia SP ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination Hostacerin AMPS (nom INCI : ammonium polyacryldimethyl-tauramide) ; les copolymères anioniques réticulés d'acrylamide et d'acide 2-acrylamido 2-méthylpropane sulfonique (AMPS), se présentant sous la forme d'une émulsion E/H, tels ceux commercialisés sous le nom de SEPIGEL 305 (nom C.T.F.A. : Polyacrylamide /C13-14 Isoparaffin / Laureth-7) et sous le nom de SIMULGEL 600 (nom C.T.F.A. : Acrylamide / Sodium acryloyldimethyltaurate copolymer / Isohexadecane /Polysorbate 80) par la société Seppic ; les polysaccharides comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose muicrocristalline, la carboxyméthylcellulose, l'hydroxyméthylcellullose et l'hydroxypropylcellulose ; et leurs mélanges. La quantité de gélifiants dépend du but recherché. La quantité de gélifiants peut aller par exemple de 0,01 à 10 %, de préférence de 0,05 à 5 % en poids et mieux de 0,05 à 3 % en poids par rapport au poids total de la composition.

Selon un mode préféré de réalisation de l'invention, la composition contient au moins un polymère choisi parmi les polymères carboxyvinyliques (carbomer), les polymères carboxyvinyliques comportant au moins un groupe hydrophobe (polymères carboxyvinyliques modifiés) (nom INCI : Acrylates/C10-30 akyl acrylate crosspolymer), les polysaccharides (notamment gomme de xanthane), les polyacrylates de sodium réticulés, les copolymères d'acide 2-acrylamido 2-méthylpropane sulfonique, et leurs mélanges.

Comme actifs utilisables dans la composition de l'invention, on peut citer en particulier les actifs antiséborrhéïques et antimicrobiens; permettant notamment de traiter les peaux grasses. Cet actif peut notamment être choisi parmi les dérivés de bêta-lactame, les dérivés de quinolone, la ciprofloxacine, la norfloxacine, la tétracycline et ses sels, l'érythromycine et ses sels, l'amikacine et ses sels, le 2,4,4'-trichloro-2'-hydroxy diphényl éther (ou triclosan), le 3,4,4'-trichlorocarbanilide (ou triclocarban), le phénoxyéthanol, le phénoxypropanol, le phénoxyisopropanol, la doxycycline et ses sels, la capréomycine et ses sels, la chlorhexidine et ses sels, la chlortétracycline et ses sels, l'oxytétracycline et ses sels, la clindamycine et ses sels, l'éthambutol et ses sels, l'hexamidine iséthionate, le métronidazole et ses sels, la pentamidine et ses sels, la gentamicine et ses sels, la kanamycine et ses sels, la lincomycine et ses sels, la méthacycline et ses sels, la méthénamine et ses sels, la minocycline et ses sels, la néomycine et ses sels, la netilmicine et ses sels, la paromomycine et ses sels, la streptomycine et ses sels, la tobramycine et ses sels, le miconazole et ses sels, les sels d'amantadine, le para-chloro-méta-xylénol, la nystatine, le tolnaftate, l'acide salicylique et ses sels, l'acide n-octanoyl-5 salicylique et ses sels, le peroxyde de benzoyle, l'acide 3-hydroxy benzoïque, l'acide 4-hydroxy benzoïque, l'acide acétylsalicylique, l'acide 2-hydroxybutanoïque, l'acide 2- hydroxy-pentanoïque, l'acide 2-hydroxyhexanoïque, l'acide phytique, la N-acétyl-L-cystéine, l'acide lipoïque, l'acide azélaïque, l'acide arachidonique, l'ibuprofène, le naproxène, l'hydrocortisone, l'acétaminophène, le résorcinol, l'octopirox, le chlorhydrate de lidocaïne, le clotrimazole, l'octoxyglycérine, l'octanoylglycine, le caprylylglycol, l'acide 10-hydroxy-2-décanoïque, les sels de zinc tels que le gluconate de zinc, la niacinamide ou vitamine B3 (ou vitamine PP), et leurs mélanges.

Comme autres actifs utilisables dans la composition de l'invention, on peut citer aussi les agents kératolytiques et anti-vieillissement comme les hydroxyacides (α-hydroxyacides et β-hydroxyacides) tels que l'acide lactique, l'acide glycolique, l'acide citrique, l'acide salicylique et leurs dérivés ; les huiles essentielles ; les vitamines et en particulier le rétinol (vitamine A), l'acide ascorbique (vitamine C), le tocophérol (vitamine E), la niacinamide (vitamine PP ou B3), le panthénol (vitamine B5) et leurs dérivés (esters par exemple) ; les co-enzymes et en particulier le co-enzyme Q10 ou ubiquinone ; les enzymes telles que par exemple les lipases, les protéases, les phospholipases, les cellulases, les peroxydases notamment les lactoperoxydases, les catalases, les superoxyde dismutases et les extraits végétaux contenant les enzymes précitées ; les levures telles que les *Saccharomyces Cerevisiae ;* les stéroïdes ; les anti-oxydants et anti-radicaux libres ; les hydratants tels que les polyols (glycérine, sorbitol, sucres), les hydrolysats de protéine, l'urée et les mélanges en contenant ; les amincissants comme la caféine ; les agents anti-élastase et anticollagénase ; les agents de traitement des peaux grasses ; les extraits végétaux et notamment les extraits de plancton ; les agents tenseurs ; les agents matifiants ; les antitranspirants tels que les sels d'aluminium ; et leurs mélanges.

Comme dérivés d'acide salicylique, on peut citer notamment les acides n-octanoyl-5-salicylique (nom INCI : Capryloyl Salicylic Acid), n-décanoyl-5-salicylique, n-dodécanoyl-5-salicylique, n-octyl-5-salicylique, n-heptyloxy-5-salicylique, 5-tert-octylsalicylique, 5-butoxy-salicylique, 5-éthoxy-salicylique, 5-méthoxysalicylique, 5-propoxysalicylique, 5-méthylsalicylique, 5-éthylsalicylique, 5-propylsalicylique, et leurs mélanges. Ces acides peuvent être éventuellement salifiés par une base.

Comme exemples de stéroïdes, on peut citer par exemple la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

La composition de l'invention peut contenir en outre des charges en vue de modifier la texture de la composition. Comme charges qui peuvent être utilisées dans la composition de l'invention, on peut citer par exemple, outre les pigments, la silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 20% en poids et de préférence de 1 à 10 % en poids par rapport au poids total de la composition.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions de l'invention peuvent constituer notamment des produits de démaquillage et/ou de nettoyage de la peau, des lèvres et/ou des yeux.

L'invention a ainsi pour objet l'utilisation cosmétique de la composition telle que définie ci-dessus, pour le démaquillage et/ou le nettoyage de la peau, des lèvres et/ou des yeux.

La composition de l'invention peut tout particulièrement être utilisée pour le démaquillage ou le nettoyage.

Aussi, la présente invention a encore pour objet un procédé cosmétique de démaquillage et/ou de nettoyage de la peau, des lèvres et/ou des yeux, caractérisé par le fait que l'on applique sur la peau, les lèvres et/ou les yeux, une composition telle que définie ci-dessus.

Par ailleurs, quand les compositions selon l'invention ont la fluidité appropriée et se présentent par exemple sous forme de lait, elles peuvent servir aussi pour l'imprégnation de substrats insolubles dans l'eau pour constituer des articles (tels que lingettes) destinés au nettoyage et/ou au démaquillage de la peau, des cils et/ou des lèvres. Le substrat insoluble dans l'eau peut comprendre une ou plusieurs couches et il peut être choisi dans le groupe comprenant des matériaux tissés, des matériaux non-tissés, des mousses, des éponges, des ouates, en feuilles, boules ou films. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés.

Quand le substrat est un non-tissé, on utilise de préférence un non-tissé épais, qui ne se met pas en boule et qui est assez solide pour ne pas se déliter et ne pas pelucher à l'application sur la peau. Il doit être absorbant, doux au moins sur une face pour le démaquillage des yeux en particulier. Comme non-tissés appropriés, on peut citer par exemple ceux commercialisés sous les dénominations Ultraloft 15285-01, Ultraloft 182-008, Ultraloft 182-010, Ultraloft 182-016 par la société BBA, Vilmed M1519 Blau, Vilmed M 1550 N et 112-132-3 par la société Freudenberg, celui commercialisé sous la dénomination Norafin 11601-010B par la société Jacob Holm Industries, les non tissés flockés commercialisés sous les dénominations Univel 109 et Univel 119 par la société Uni Flockage.

Par ailleurs, ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

L'invention a donc aussi pour objet un article obtenu par imprégnation d'un substrat insoluble dans l'eau, avec une composition telle que définie ci-dessus.

L'invention a aussi pour objet l'utilisation de la composition telle que définie ci-dessus pour la préparation d'un article destiné au démaquillage ou au nettoyage de la peau, des lèvres et/ou des cils.

Les exemples ci-après de compositions selon l'invention sont donnés à titre d'illustration et sans caractère limitatif. Les quantités y sont données en % en poids, sauf mention contraire.

**Exemple 1 selon l'invention : lait de démaquillage**

| | |
|---|---|
| *Phase A* - Glycérine | 3 % |
| - Conservateurs | 0,2 % |
| - Dimethicone copolyol (DC 5225 C) (soit 0,15 % de matière active) | 1,5 % |
| - Disodium cocoamphodiacétate | 1 % |
| - Sodium laureth sulfate | 1 % |
| - Pemulen TR2 | 0,15 % |
| - Eau | qsp 100 % |

| | |
|---|---|
| *Phase B.* | |
| - Palmitate d'éthyl hexyle | 15 % |

| | |
|---|---|
| *Phase C.* | |
| - Hydroxyde de sodium (NaOH) | qsp pH 6,5 |
| - Eau | 5 % |

Mode opératoire : On prépare la phase A en chauffant le mélange des composés, sans le Pemulen, à 65°C - 70 °C, puis on ajoute le Pemulen en le dispersant sous cisaillement, et on ajoute ensuite la phase B sous agitation puis la phase C.

On obtient un lait blanc souple, homogène, lisse et brillant qui est agréable à utiliser et a une bonne efficacité de démaquillage.

**Exemple 2 comparatif**

*Phase A*

| | |
|---|---|
| - Glycérine | 3 % |
| - Conservateurs | 0,2 % |
| - Disodium cocoamphodiacétate | 1 % |
| - Sodium laureth sulfate | 1 % |
| - Pemulen TR2 | 0,15 % |
| - Eau | qsp 100 % |

*Phase B.*

| | |
|---|---|
| - Palmitate d'éthyl hexyle | 15 % |

*Phase C.*

| | |
|---|---|
| - hydroxyde de sodium (NaOH) | qsp pH 6,5 |
| - Eau | 5 % |

Mode opératoire : Comme dans l'exemple 1

On obtient un lait souple et homogène mais qui a une moins bonne efficacité de démaquillage.

**Exemple 3 comparatif**

*Phase A*

| | |
|---|---|
| - Glycérine | 5 % |
| - Conservateurs | 0,2 % |
| - Disodium cocoamphodiacétate | 1 % |
| - Sodium laureth sulfate | 1 % |
| - Gomme de xanthane | 0,1 % |
| - carbomer | 0,5 % |
| - Eau | qsp 100 % |

*Phase B.*

| | |
|---|---|
| - huile de vaseline | 12 % |
| - Palmitate d'ethyl hexyle | 7 % |
| - Alcool cétéarylique | 0,4 % |

*Phase C.*

| | |
|---|---|
| - hydroxyde de sodium (NaOH) | qsp pH 6,5 |
| - Eau | 5 % |

Mode opératoire : comme dans l'exemple 1

On obtient un lait lisse et souple mais qui a une moins bonne efficacité de démaquillage.

Test de démaquillage : un test a été réalisé pour comparer l'efficacité de démaquillage de l'exemple 1 selon l'invention par rapport aux exemples comparatifs 2 et 3.

I. Protocole de démaquillage in Vivo

I.a. Matériel

1. Huile démaquillante rinçable à l'eau : « Huile Démaquillante fraîche » de Shu Uemura
2. Savon de Marseille
3. 1 cache comportant une partie centrale vide découpée dans une fiche bristol de 4x4 cm + 1 feutre LUMICOLOR permanent S
4. Colorimètre CR300 (mesure de la colorimétrie en L.a.b)
5. Fond de teint sans transfert Air Wear « Sable » LSF 12 de L'Oreal
6. 8 petits verres de montre + des petites spatules
7. 1 balance de précision
8. 1 chronomètre

I.b. Protocole

- On trace sur la peau 4 zones (2 par bras) de la dimension de la partie centrale découpée du cache, à l'aide du feutre et du cache ;
- On nettoie la peau de ces zones à l'huile démaquillante rinçable puis au savon, on rince et on sèche (les marques de feutre sont alors atténuées pour éviter tout transfert de feutre dans le fond de teint au moment de l'application) ;
- On mesure par colorimétrie la peau nue : 3 mesures de L,a,b par zone (tête de lecture du colorimètre positionnée parfaitement au centre de la zone),
- On applique dans les 4 zones délimitées, de façon homogène, 16 mg+/- 0,05 mg de fond de teint et on attend 30 minutes ;
- On mesure par colorimétrie la peau avec fond de teint : 3 mesures de L,a,b par zone ;
- On pèse dans les verres de montre 4 fois, 208 mg+/-1 mg de composition démaquillante à tester ;
- On démaquille chaque zone au doigt par mouvement circulaire pendant 10 secondes puis on rince à l'eau du robinet (tiède ; dureté non contrôlée) en effleurant légèrement avec les doigts de la main disponible. On fait les 4 zones sans attendre.
- On tamponne avec un mouchoir jetable type kleenex et on attend 15 minutes (sensation de peau sèche) ;
- On mesure par colorimétrie la peau démaquillée : 3 mesures de L,a,b par zone.

Le calcul du pourcentage de démaquillage est fait de la manière suivante :

- On détermine l'écart colorimétrique de la peau maquillée (peau FdT) par rapport à la peau nue, ΔEmax étant la valeur correspondant à un démaquillage parfait :

$$\Delta E \, max = \sqrt{(\Delta a_1^2 + \Delta b_1^2 + \Delta L_1^2)}$$

avec

$\Delta a_1$ = a peau nue - a peau FdT
$\Delta b_1$ = b peau nue - b peau FdT
$\Delta L_1$ = L peau nue - L peau FdT

- On détermine pour la composition démaquillante testée, l'écart colorimétrique ΔE de la peau démaquillée (peau démaq) par rapport à la peau maquillée (peau FdT) pour chaque zone :

$$\Delta E = \sqrt{(\Delta a_2^2 + \Delta b_2^2 + \Delta L_2^2)}$$

avec

$\Delta a_2$ = a peau démaq - a peau FdT
$\Delta b_2$ = b peau démaq - b peau FdT
$\Delta L_2$ = L peau démaq - L peau FdT

- On détermine le rapport ΔE/ΔEmax.
- on calcule le pourcentage de démaquillage moyen qui est la moyenne des 4 valeurs de (ΔE/ΔEmax) x 100 pour la composition démaquillante testée.

Le tableau suivant donne les résultats de l'exemple 1 de l'invention et des exemples comparatifs 2 et 3 :

| Composition démaquillante | Taux d'huile démaquillante | Efficacité démaquillante |
|---|---|---|
| Exemple 1 selon l'invention | 15% | 43% |
| Exemple 2 comparatif | 15% | 26% |
| Exemple 3 comparatif | 19% | 19% |

La comparaison des exemples 1 et 3 montre que l'efficacité démaquillante est meilleure pour l'exemple 1 selon l'invention qui contient pourtant moins d'huile que l'exemple comparatif 3.

La comparaison des exemples 1 et 2 montre que l'efficacité démaquillante est meilleure pour l'exemple 1 qui ne diffère de l'exemple 2 que par l'ajout de 1,5% de DC 5225 C.

**Exemple 4 selon l'invention : crème de démaquillage**

*Phase A*

| | |
|---|---|
| - Glycérine | 3 % |
| - Conservateurs | 0,3 % |
| - Disodium cocoamphodiacétate | 0,75 % |
| - Sodium laureth sulfate | 0,75 % |
| - Eau | qsp 100 % |

*Phase B.*

| | |
|---|---|
| - Palmitate d'éthyl hexyle | 20 % |
| - Huile de Vaseline | 15 % |
| - Petrolatum | 20 % |

*Phase C.*

| | |
|---|---|
| - Huile de vaseline | 5 % |
| - Carbomer | 0,7 % |

Phase D

| | |
|---|---|
| - Dimethicone copolyol (DC 5225 C) (soit 0,07 % de matière active) | 0,7 % |

Phase E

| | |
|---|---|
| - Eau | 1,5 % |
| -Triéthanolamine | 0,8 % |

Mode opératoire : on chauffe la phase A à 75°C, puis on ajoute la phase B puis la phase C dans la phase A sous agitation pour former l'émulsion. On ajoute ensuite la phase D sous agitation. Enfin, on neutralise par ajout de la phase E.

On obtient une crème ayant une viscosité d'environ 40 poises à 25°C. C'est une crème blanche, lisse, brillante et onctueuse, qui peut être efficacement utilisée pour le démaquillage de la peau.

**Revendications**

1.  Composition cosmétique sous forme d'émulsion huile-dans-eau, comportant une phase huileuse dispersée dans une phase aqueuse, **caractérisée en ce qu'**elle comprend au moins un tensioactif siliconé, au moins une huile et au moins un tensioactif détergent.

2.  Composition selon la revendication 1, **caractérisée en ce que** le tensioactif siliconé est choisi parmi les dimethicone copolyols et les alkyldimethicone copolyols.

3.  Composition selon la revendication 1 ou 2, **caractérisée en ce que** le tensioactif siliconé comporte de 2 à 50 groupes oxyéthylénés et de 2 à 50 groupes oxypropylénés.

4.  Composition selon la revendication 1 ou 2, **caractérisée en ce que** le tensioactif siliconé est choisi parmi le dimethicone copolyol comportant 18 groupes oxyéthylénés et 18 groupes oxypropylénés, le dimethicone copolyol comportant 14 groupes oxyéthylénés et 14 groupes oxypropylénés, le cétyl diméthicone copolyol, le lauryl diméthicone copolyol et leurs mélanges.

5.  Composition selon l'une quelconque des revendications précédentes, **caractérisée en ce que** la quantité de tensioactif siliconé va de 0,01 à 5 % en poids par rapport au poids total de la composition.

6.  Composition selon l'une quelconque des revendications précédentes, **caractérisée en ce que** la phase huileuse constitue de 3 à 70 % en poids par rapport au poids total de la composition.

7.  Composition selon l'une quelconque des revendications précédentes, **caractérisée en ce que** l'huile est une huile démaquillante choisie parmi les huiles hydrocarbonées, les esters gras comportant au moins 8 atomes de carbone, et leurs mélanges

8.  Composition selon l'une quelconque des revendications précédentes, **caractérisée en ce que** la quantité de tensioactif(s) détergent(s) va de 0,05 à 10 % en poids par rapport au poids total de la composition.

9.  Composition selon l'une quelconque des revendications précédentes, **caractérisée en ce que** le tensioactif détergent a un HLB égal ou supérieur à 11.

10. Composition selon l'une quelconque des revendications précédentes, **caractérisée en ce que** le tensioactif détergent est choisi parmi les tensioactifs détergents non ioniques, anioniques, amphotères et zwitterioniques et leurs mélanges.

11. Composition selon l'une quelconque des revendications précédentes, **caractérisée en ce que** le tensioactif détergent est choisi parmi les alkylpolyglucosides, les alkyl éther sulfates, les N-acylglutamates, les acyliséthionates, les sulfosuccinates, les phosphates et alkylphosphates, les bétaïnes, les alkylamphoacétates, et leurs mélanges.

12. Composition selon l'une quelconque des revendications précédente, **caractérisée en ce qu'**elle contient au moins un polymère choisi parmi les polymères carboxyvinyliques, les polymères carboxyvinyliques comportant au moins un groupement hydrophobe, les polysaccharides, les polyacrylates de sodium réticulés, les polymères d'acide 2-acrylamido 2-méthylpropane sulfonique, et leurs mélanges.

13. Utilisation cosmétique de la composition selon l'une quelconque des revendications 1 à 12 pour le démaquillage et/ou le nettoyage de la peau, des lèvres et/ou des yeux.

14. Procédé cosmétique de démaquillage et/ou de nettoyage de la peau, des lèvres et/ou des yeux, **caractérisé en ce que** l'on applique sur la peau, les lèvres et/ou les yeux, une composition selon l'une quelconque des revendications 1 à 12.

15. Utilisation cosmétique d'au moins un tensioactif siliconé pour améliorer l'efficacité de démaquillage d'une émulsion huile-dans-eau contenant au moins une huile et au moins un tensioactif détergent.

16. Article obtenu par imprégnation d'un substrat insoluble dans l'eau, avec une composition selon l'une quelconque des revendications 1 à 12.

**17.** Utilisation de la composition selon l'une quelconque des revendications 1 à 12, pour la préparation d'un article destiné au soin, au démaquillage, au nettoyage ou au maquillage de la peau, des lèvres et/ou des cils.

**Office européen**

**des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | WO 01/58419 A (RHODIA CHIMIE ET AL.) 16 août 2001 (2001-08-16) * exemple 1 * * page 5, ligne 36 - page 7, ligne 23 * ----- | 1-17 | INV. A61K8/06 A61K8/891 A61Q1/14 |
| Y | EP 1 405 634 A (L'OREAL) 7 avril 2004 (2004-04-07) * exemples 1-3 * * alinéas [0001], [0038] - [0040], [0043], [0044] * ----- | 1-17 | |
| Y | EP 1 466 587 A (L'OREAL) 13 octobre 2004 (2004-10-13) * revendications 1,7,9,11,12 * * alinéas [0001], [0026], [0030], [0032], [0041], [0042], [0046] - [0048], [0056] - [0059] * ----- | 1-17 | |
| A | EP 1 093 844 A (L'OREAL) 25 avril 2001 (2001-04-25) * revendication 1; exemple 3 * * alinéas [0001], [0002], [0007] * ----- | 1-17 | DOMAINES TECHNIQUES RECHERCHES (IPC) A61K |

Le présent rapport a été établi pour toutes les revendications

| Lieu de la recherche | Date d'achèvement de la recherche | Examinateur |
|---|---|---|
| Berlin | 11 mai 2006 | Alvarez Alvarez, C |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

.....................................................................
& : membre de la même famille, document correspondant

EPO FORM 1503 03.82 (P04C02)

**EP 1 695 690 A1**

**ANNEXE AU RAPPORT DE RECHERCHE EUROPEENNE
RELATIF A LA DEMANDE DE BREVET EUROPEEN NO.**

EP 05 02 7826

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-05-2006

| Document brevet cité au rapport de recherche | | Date de publication | Membre(s) de la famille de brevet(s) | | Date de publication |
|---|---|---|---|---|---|
| WO 0158419 | A | 16-08-2001 | AU | 2555000 A | 20-08-2001 |
| EP 1405634 | A | 07-04-2004 | FR<br>JP | 2844707 A1<br>2004269492 A | 26-03-2004<br>30-09-2004 |
| EP 1466587 | A | 13-10-2004 | FR<br>JP | 2853527 A1<br>2005097243 A | 15-10-2004<br>14-04-2005 |
| EP 1093844 | A | 25-04-2001 | FR<br>JP | 2799989 A1<br>2001158715 A | 27-04-2001<br>12-06-2001 |

EPO FORM P0460